# EUROPEAN PATENT SPECIFICATION

(11) **EP 3 270 981 B1**
(45) Date of publication and mention of the grant of the patent: **22.11.2023**
(21) Application number: 16713160.6
(22) Date of filing: 02.03.2016
(51) Int. Cl.: A61L 9/03

(54) **SYSTEM AND METHOD FOR DISPENSING MATERIAL**
SYSTEM UND VERFAHREN ZUR AUSGABE EINES MATERIALS
SYSTÈME ET PROCÉDÉ DE DISTRIBUTION DE MATIÈRE

(30) Priority: 16.03.2015 US 201514658280
(43) Date of publication of application: 24.01.2018
(73) Proprietor: The Procter & Gamble Company, Cincinnati, OH 45202 (US)
(72) Inventor: BUSH, Stephan, Gary, Cincinnati, Ohio 45202 (US); SHERMAN, Faiz, Feisal, Cincinnati, Ohio 45202 (US); GRUENBACHER, Dana, Paul, Cincinnati, Ohio 45202 (US)
(74) Representative: P&G Patent Belgium UK
(86) International application number: PCT/US2016/020317
(87) International publication number: WO 2016/148912

(56) References cited:
- EP-A1- 1 510 228
- EP-A2- 1 894 727
- US-A1- 2009 096 839
- US-A1- 2009 126 722

## Description

### FIELD OF THE INVENTION

The invention relates to a method for dispensing materials. The invention relates particularly to a method for dispensing materials by using a thermal jetting system.

### BACKGROUND OF THE INVENTION

Dispensing materials via the heating of a volatile carrier to form a transport jet is well known. Thermal ink-jet systems provide a means for the creation and precise deposition of ink droplets upon a substrate. Thermal driven systems may also be used to drive the dispensing or dispersion of other materials, again by volatilizing a carrier or the actual material to be dispensed.

The 'atomization' of volatile oils to disperse them in an environment for the purpose of spreading a fragrance in the environment is also known. Typical dispersion systems create a set of oil droplets which disperse in the environment. Many such oil droplets rapidly settle out of the atmosphere of the environment resulting in an oily deposit upon surfaces, and more significantly, a reduction in the concentration of the fragrance oils in the environment's atmosphere.

What is needed is an improved system and method for the dispersion of materials into an environment such that the material may be easily dispersed in the environment and may also remain airborne for a longer period of time.

US2009096839 discusses a fluid ejection device; US2009126722 discusses a liquid ejection device; EP1510228 discusses a scent delivery apparatus; and EP1894727 discusses a liquid ejecting head.

### SUMMARY OF THE INVENTION

The invention provides a liquid dispensing method in accordance with the claims.

### BRIEF DESCRIPTION OF THE DRAWINGS

Figure 1 provides a schematic representation of a device useable for the method of the invention.
Figure 2 provides a schematic sectional representation of a chamber of a device useable for the method of the invention, taken along section line AA of Figure 1.

### DETAILED DESCRIPTION OF THE INVENTION

The present disclosure is directed to a fluid delivery system configured to eject a working fluid from a thermal micro-fluidic die. The die includes one or more heating elements formed in a substrate. The substrate may be covered by a dielectric layer, shielding the heating elements from direct contact with the working fluid intended for ejection by the system. Each heating element is disposed adjacent to a fluid chamber and in turn to a nozzle disposed adjacent to the fluid chamber. Well known structures associated with electronic components and the details of semiconductor fabrication have not been included in the description.

In one aspect, the method of the invention employs an apparatus for dispensing a liquid into an environment. The apparatus comprises a reservoir containing the liquid. The reservoir is in fluid communication with a dispensing element or print head. The connection between the reservoir and the dispensing element enables the fluid to flow from the reservoir to flow into chambers of the dispensing element. The chambers are generally a rectangular prism and have a height (H), length (L) and width (W). One surface of the chamber includes an activation element of length L2. Each chamber is disposed adjacent to an orifice, or nozzle, which in turn is open to the external environment of the apparatus. In one embodiment, the ratio of the nozzle diameter D to the chamber height H is between about 3 and about 10. The ratio of the length L2 of the activation element to the chamber height H may be between about 3 and about 11.

Selective manipulation of the ratio of the length L2 to the chamber height H, and/or the ratio of the diameter D to the chamber height H in the design and fabrication of the chamber, die and nozzle elements may advantageously enable operating conditions for the system. Such conditions include the ability to discharge fluid from the nozzles at rates in excess of the ability of the fluid to completely refill the chambers. A consequence of this mode of operation is that the droplets of fluid discharged from the nozzles may be smaller than the nozzle diameter D, which may be an operational advantage when small drops are desired but small nozzle diameter is undesired or impractical.

A control element is disposed in electrical communication with the activation elements and enables the application of energy to the liquid by the activation elements.

Liquid disposed within the chamber may be selectively volatilized by the transfer of energy from the activation element to the liquid. The activation element may comprise a resistive heating element or a piezoelectric element. Upon the transfer of sufficient energy to the liquid, (for example in the case of thermal activation) at least a portion of the liquid will become superheated and will vaporize. The liquid within the chamber will be ejected via the nozzle into the external environment as one or more droplets.

The frequency at which the activation element imparts energy pulses to the liquid within the chamber exceeds the frequency with which the liquid can physically refill the chamber. The activation element fires and activates the liquid before the chamber is completely filled by the liquid. In one embodiment, the activation elements may be fired at a frequency of greater than 5000Hz for chambers having dimensions D~30um, H~10um, and L~50um, with a fluid having surface tension of about 30 mN/m and viscosity of about 7 mPa·s.

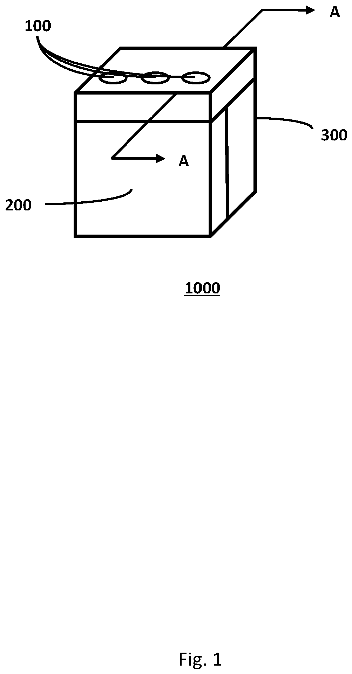

As illustrated in Figure 1, the device 1000, comprises a plurality of dispensing elements 100, a fluid reservoir 300 in communication with the dispensing elements, and a control element 200 in electrical communication with the dispensing elements.

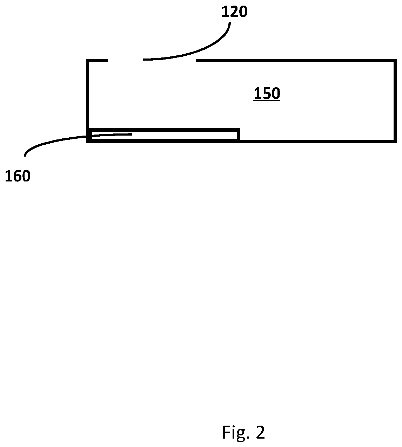

Figure 2 provides a sectional view of a portion of Figure 1 taken along line AA, showing the interior structure of a dispensing element 100 having a chamber 150, a nozzle, 120 and an activation element 160.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A liquid dispensing method, the method comprising steps of:
providing a dispensing device, the device comprising:
a plurality of liquid dispensing elements (100), each element comprising a chamber (150) that is generally a rectangular prism and which has a height (H), length (L) and width (W), a nozzle having a diameter (D), and an activation element (160) having a length (L2) and a refill time (RT),
a liquid containing reservoir (300) in fluid communication with the liquid dispensing elements;
a liquid contained in the liquid containing reservoir (300);
a control element (200) in electrical communication with the liquid dispensing elements; wherein the ratio of the nozzle diameter (D) to chamber height (H) is between 3 and 10;
flowing fluid from the reservoir (300) into the chambers (150) via a connection between the reservoir (300) and the dispensing elements (100); and
repeatedly energizing the activation elements at a frequency greater than 1/(RT) whereby the activation element fires and activates the liquid prior to the chambers being completely filled by the liquid.

2. The method according to claim 1 wherein the ratio of the activation element length (L) to the chamber height (H) is between 3 and 11.

## Patentansprüche

1. Flüssigkeitsabgabeverfahren, das Verfahren umfassend die Schritte:
Bereitstellen einer Abgabevorrichtung, die Vorrichtung umfassend:
eine Vielzahl von Flüssigkeitsabgabeelementen (100), jedes Element umfassend eine Kammer (150), die im Allgemeinen ein rechteckiges Prisma ist und die eine Höhe (H), eine Länge (L) und eine Breite (W) aufweist, eine Düse, die einen Durchmesser (D) aufweist, und ein Aktivierungselement (160), das eine Länge (L2) aufweist, und eine Nachfüllzeit (RT),
ein Flüssigkeit enthaltendes Reservoir (300), das in Fluidaustausch mit den Flüssigkeitsabgabeelementen steht;
eine Flüssigkeit, die in dem Flüssigkeit enthaltenden Reservoir (300) enthalten ist;
ein Steuerelement (200), das in elektrischem Austausch mit den Flüssigkeitsabgabeelementen steht; wobei das Verhältnis des Düsendurchmessers (D) zu der Kammerhöhe (H) zwischen 3 und 10 ist;
Leiten von Fluid aus dem Reservoir (300) in die Kammern (150) über eine Verbindung zwischen dem Reservoir (300) und den Abgabeelementen (100); und
wiederholtes Erregen der Aktivierungselemente bei einer Frequenz größer als 1/(RT), wobei das Aktivierungselement die Flüssigkeit feuert und aktiviert, bevor die Kammern vollständig durch die Flüssigkeit gefüllt werden.

2. Verfahren nach Anspruch 1, wobei das Verhältnis der Aktivierungselementlänge (L) zu der Kammerhöhe (H) zwischen 3 und 11 liegt.

## Revendications

1. Procédé de distribution de liquide, le procédé comprenant les étapes consistant à :
fournir un dispositif de distribution, le dispositif comprenant :
une pluralité d'éléments de distribution de liquide (100), chaque élément comprenant une chambre (150) qui est généralement un prisme rectangulaire et qui a une hauteur (H), une longueur (L) et une largeur (W), une buse ayant un diamètre (D), et un élément d'activation (160) ayant une longueur (L2) et un temps de recharge (RT),
un réservoir contenant un liquide (300) en communication fluidique avec les éléments de distribution de liquide ;
un liquide contenu dans le réservoir contenant un liquide (300) ;
un élément de commande (200) en communication électrique avec les éléments de distribution de liquide ; dans lequel le rapport du diamètre de buse (D) à la hauteur de chambre (H) est compris entre 3 et 10 ;
écouler le fluide depuis le réservoir (300) dans les chambres (150) par l'intermédiaire d'un raccord entre le réservoir (300) et les éléments de distribution (100) ; et
exciter de manière répétée les éléments d'activation à une fréquence supérieure à 1/(RT) moyennant quoi l'élément d'activation déclenche et active le liquide avant que les chambres ne soient complètement remplies par le liquide.

2. Procédé selon la revendication 1, dans lequel le rapport de la longueur d'élément d'activation (L) à la hauteur de chambre (H) est compris entre 3 et 11.
